(19) Europäisches Patentamt
European Patent Office
Office européen des brevets

(11) **EP 3 558 158 B1**

(12) **EUROPEAN PATENT SPECIFICATION**

(45) Date of publication and mention
of the grant of the patent:
**05.05.2021 Bulletin 2021/18**

(21) Application number: **17821546.3**

(22) Date of filing: **15.12.2017**

(51) Int Cl.:
*A61C 17/22* (2006.01)          *A61C 1/14* (2006.01)
*A46B 5/00* (2006.01)          *A61C 15/04* (2006.01)
*B29C 65/02* (2006.01)

(86) International application number:
**PCT/EP2017/082956**

(87) International publication number:
**WO 2018/114638 (28.06.2018 Gazette 2018/26)**

(54) **COUPLING FOR PERSONAL CARE DEVICE**

VERBINDUNG FÜR KÖRPERPFLEGEVORRICHTUNG

CONNECTION POUR DISPOSITIF DE SOINS PERSONNELS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.12.2016 US 201662436680 P
31.03.2017 EP 17164232**

(43) Date of publication of application:
**30.10.2019 Bulletin 2019/44**

(73) Proprietor: **Koninklijke Philips N.V.
5656 AG Eindhoven (NL)**

(72) Inventors:
• **VAN DEN ENDE, Daan, Anton
5656 AE Eindhoven (NL)**
• **LAVEZZO, Valentina
5656 AE Eindhoven (NL)**

(74) Representative: **Schudelaro, Antonius Adrianus
Petrus
Philips Intellectual Property & Standards
High Tech Campus 5
5656 AE Eindhoven (NL)**

(56) References cited:
**EP-A1- 0 685 209          WO-A1-2009/155718
US-A1- 2004 088 835          US-A1- 2008 028 587
US-A1- 2010 298 851          US-A1- 2013 125 921**

**EP 3 558 158 B1**

**Description**

TECHNICAL FIELD

The present embodiments relate generally to personal care devices, such as power toothbrushes, and more particularly, to components for a personal care device, such as a toothbrush head and handle portion.

BACKGROUND

WO 2009/155718 discloses an implantation device comprises an implantation tool, or an intermediate piece that can be connected to a tool, and an implant that can be coupled or is coupled to the tool or intermediate piece. For the purpose of this coupling, the tool or intermediate piece and the implant have connecting structures cooperating for a positive or non-positive connection, wherein one of the connecting structures is equipped with a shape memory element. At ambient temperatures and body temperatures, the connecting structures have a connecting configuration. Heating or cooling brings about a shape change of the shape memory element such that the connecting structures are transferred into a non-connecting configuration. For the purpose of implantation, the implant is coupled to the tool or intermediate piece. After implantation, the shape memory element is heated or cooled in situ, and the tool or intermediate piece is decoupled and removed from the implant.

US 2010/0298851 discloses a way to couple a surgical tool to a waveguide. The clinician positions a distal end portion of the waveguide into a cavity of the surgical tool. Thermal energy (i.e., heat) is then applied to the distal end portion of the waveguide to increase the outside diameter or parametrical shape of the distal end portion through thermal expansion. The heat or thermal energy may be applied to the waveguide by a radio frequency (RF) induction coil mounted about the waveguide adjacent the distal end portion. In other embodiments, a resistive thermoelectric heat element may be employed. Heat is applied until a sufficient interference fit is established between the proximal end portion of the surgical tool and the distal end portion of the waveguide. Thereafter, the heat applicator must continue to be energized to maintain the interference fit throughout use. After the surgical procedure has been completed, the heat applicator may be de-energized. Once the temperature of the proximal end portion of the waveguide returns to the approximate temperature of the proximal end of the surgical tool, the surgical tool may be detached from the waveguide.

US 2013/0125921 discloses a makeup applicator with a multiplicity of interchangeable heads. Each head includes a spring which reversibly engages a ferrule, enabling the substitution of applicator heads on a single handle-ferrule assembly. In some embodiments, the handle further comprises an attachment mechanism whereby the ferrule is removably attached to the handle. In one embodiment, the spring on each applicator head further comprises a protrusion on its outer surface; and said ferrule further comprises a groove on its inner surface for reversibly engaging said protrusion. In other embodiments, an energy such as electrical, microwave radiation, infrared radiation, or heat is applied to partially melt the handle or ferrule at the interface, such that the melting and subsequent cooling of the interface will form an attachment.

In conjunction with oral healthcare, a primary principle of plaque removal with a toothbrush (e.g., a power toothbrush) is to get sufficient bristle tip pressure to the toothbrush head. Optimizing power transfer from the motor to the brush head is one mechanism for achieving this goal. Similar goals of maximizing power transfer efficiency are also desired with other types of personal care devices such as electric shaving devices.

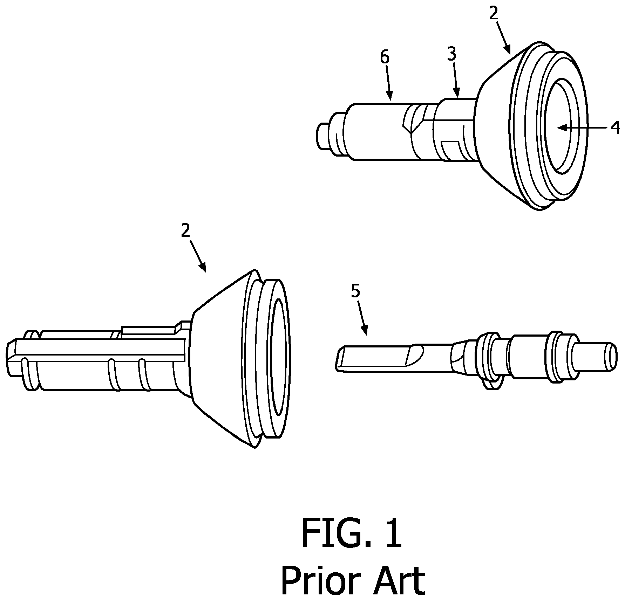

Power toothbrushes have replaceable heads which can be clicked on to and off from the handle. Current designs have a mechanical coupling mechanism similar to that shown in Fig. 1. The toothbrush head 2 has a body 3 with a coupling receptacle or cavity 4 that is shaped to receive a portion of the drive shaft 5 of the handle. A preload spring 6 may also be included to provide the required preload to ensure adequate torque transfer from the drive shaft 5 to the brush neck and creates the normal force required for the friction interface for axial retention.

Generally, a toothbrush head must be coupled to a toothbrush handle by a mechanism that both allows easy removal and replacement, while preventing the toothbrush head from falling or vibrating off while in use. However, it must not be loose and as a result rattle and/or amplify vibrations that impact on the user experience. This involves compromises in design, in particular the clearance tolerances allowed between a drive shaft of the handle of the toothbrush, and the corresponding fitting of the toothbrush head. There must be sufficient clearance that the two parts are easily coupled and separated over all parts made, but if the tolerances are too large, then there will be rattling during use while the drive shaft is imparting drive forces to the brush head, enabling the cleaning action. This rattling can detract from the user experience of using the toothbrush. In addition, too large clearances result in inefficient power and motion transfer from the handle to the brush head.

Furthermore, for some users such as the elderly, the force needed to attach and detach toothbrush heads is too high and it would be desirable to have a mechanism with lower attachment and detachment forces, whilst retaining sufficient clamping force during use of the device to prevent the attachment from falling off due to vibrations.

Similar considerations apply to other types of personal care devices in which two components are to be coupled together and driving forces transferred from one component to the other, for example tongue cleaners, shavers, hair clippers or trimmers, hair removal devices, or skin care devices, etc.

SUMMARY

Thus, in view of the above problems, improvements to coupling mechanisms in personal care devices and components for personal care devices are desired. The invention is defined by the independent claims. The dependent claims define advantageous embodiments.

These and other aspects of the invention will be apparent from and elucidated with reference to the embodiments described hereinafter.

BRIEF DESCRIPTION OF THE DRAWINGS

The embodiments of the present disclosure may take form in various components and arrangements of components, and in various steps and arrangements of steps. Accordingly, the drawings are for purposes of illustrating the various embodiments and are not to be construed as limiting the embodiments, but to understand the present invention which is defined by the scope of the appended claims. . In the drawing figures, like reference numerals refer to like elements. In addition, it is to be noted that the figures may not be drawn to scale.

   Fig. 1 is a perspective view of an attachment structure of the prior art;
   Fig. 2 is a cross-sectional view of a power toothbrush according to an embodiment;
   Fig. 3 is an exploded perspective view of an attachment structure in a power toothbrush head;
   Fig. 4 shows a cut-away side view illustration of an attachment structure according to an embodiment;
   Figs. 5a, 5b and 5c show three cross sections through part of an attachment structure of Fig. 4 with a drive shaft inserted;
   Figs. 6a, 6b and 6c show a coupling mechanism that uses a material having a shape memory effect;
   Figs. 7a, 7b and 7c show an example of an alternative coupling mechanism according to an embodiment;
   Fig. 8 is a diagram of a handle of a personal care device according to an alternative embodiment; and
   Fig. 9 shows two method steps of operating a personal care device.

DETAILED DESCRIPTION OF EMBODIMENTS

The embodiments of the present disclosure and the various features and advantageous details thereof are explained more fully with reference to the non-limiting examples that are described and/or illustrated in the drawings and detailed in the following description. It should be noted that the features illustrated in the drawings are not necessarily drawn to scale, and features of one embodiment may be employed with other embodiments as the skilled artisan would recognize, even if not explicitly stated herein. Descriptions of well-known components and processing techniques may be omitted so as to not unnecessarily obscure the embodiments of the present disclosure. The examples used herein are intended merely to facilitate an understanding of ways in which embodiments of the present disclosure may be practiced and to further enable those of skill in the art to practice the same. Accordingly, the examples herein should not be construed as limiting the scope of the embodiments of the present disclosure, which is defined solely by the appended claims .

It is understood that the embodiments of the present disclosure are not limited to the particular methodology, protocols, devices, apparatus, materials, applications, etc., described herein, as these may vary. It is also to be understood that the terminology used herein is used for the purpose of describing particular embodiments only, and is not intended to be limiting in scope of the embodiments as claimed. It must be noted that as used herein and in the appended claims, the singular forms "a," "an," and "the" include plural reference unless the context clearly dictates otherwise.

Unless defined otherwise, all technical and scientific terms used herein have the same meanings as commonly understood by one of ordinary skill in the art to which the embodiments of the present disclosure belong. Preferred methods, devices, and materials are described, although any methods and materials similar or equivalent to those described herein can be used in the practice or testing of the embodiments.

According to a first embodiment, there is provided a first component for a personal care device, wherein the first component comprises a body having a first coupling interface for coupling the first component to a second component for the personal care device via a second coupling interface of the second component; the first coupling interface comprising a portion of material that can change shape with changes in temperature to couple the first coupling interface to the second coupling interface. This will enable motion, vibration and/or torque to be transferred between the first component and the second component.

According to a second embodiment, there is provided a personal care device comprising a first component, wherein the first component comprises a body having a first coupling interface for coupling the first component to a second component for the personal care device via a second coupling interface of the second component; the first coupling interface comprising a portion of material that can change shape with changes in temperature to couple the first coupling interface to the second coupling interface. This will enable motion, vibration and/or torque to be transferred between the first component and the second component.

According to a third embodiment, there is provided a method of operating a personal care device, the personal care device comprising a first component and a second component, the first component comprising a body having a first coupling interface, and the second component comprising a second coupling interface, the method comprising coupling the first component to the second component by changing the temperature of a portion of material in the first coupling interface to change the shape of the portion of material and couple the first coupling interface to the second coupling interface. The personal care device can then transfer motion, vibration and/or torque between the first component and the second component.

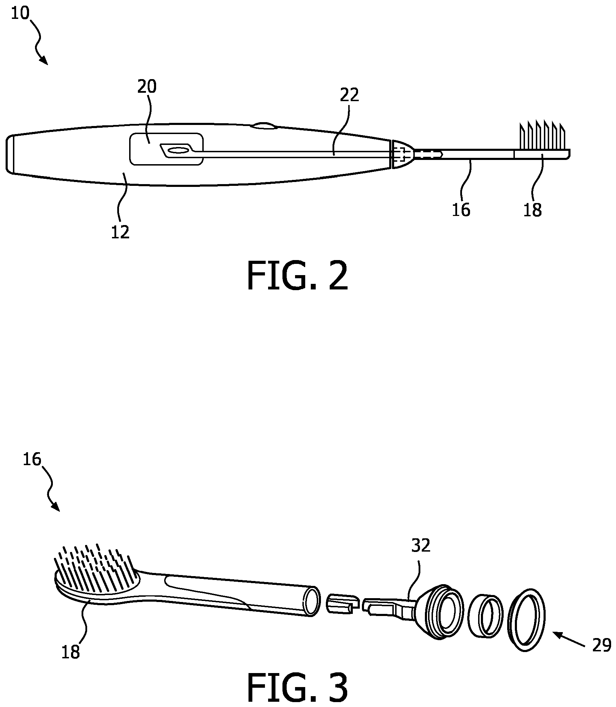

Fig. 2 shows an exemplary personal care device 10 in which the teaching of the present disclosure can be implemented. The personal care device in Fig. 2 is in the form of an electric toothbrush, but it will be appreciated that this is not limiting, and the teaching of the present disclosure can be implemented in other devices in which two components are to be coupled together, particularly where one of the components is a detachable and/or replaceable component. For example, the teachings can be applied to personal care devices such as tongue cleaners, shavers, hair clippers or trimmers, hair removal devices, or skin care devices. The personal care device 10 has an attachment structure 16 and a handle portion 12. In one embodiment, the attachment structure 16 comprises or is a replaceable attachment, i.e. the attachment structure 16 can be removed from the personal care device 10 and replaced by another attachment structure 16. The handle 12 includes a drive train/controller 20 and a drive shaft 22. The drive shaft 22 extends from a distal end of the handle 12, and into the attachment structure 16 when an attachment structure 16 is attached to the handle 12.

In embodiments, drive train/controller 20 can be configured for controlling an operation or operations of the drive train 20 to produce a mechanical stimulus and drive the draft shaft 22. The attachment structure 16 (which is also referred to herein as attachment) has extends between the handle portion at a proximal end thereof and a distal end of the attachment 16. The proximal end couples, via a coupling mechanism, to the portion of drive shaft 22 extending from the distal end of the handle 12. The distal end 30 of the attachment 16 can include a head 18, configured according to the requirements of a specific application of the attachment 16. In operation, the drive train 20 drives the drive shaft 22 to produce a mechanical stimulus that moves attachment structure 16.

Fig. 3 shows an exploded perspective view of an exemplary attachment structure 16. The attachment structure 16 is designed to couple to the portion of the drive shaft 22 that extends from the distal end of the handle 12 of the personal care device 10. The attachment structure 16 comprises a coupling mechanism 29 located at the proximal end of the attachment 16. The coupling mechanism comprises a coupling interface 32 that connects, i.e. couples, the drive shaft 22 to the attachment 16, for transferring drive motion, vibration and/or torque from drive shaft 22 to the attachment structure 16.

As noted above, coupling mechanisms in personal care devices such as power toothbrushes should allow both easy removal and replacement of attachments such as toothbrush heads, while preventing the attachment from falling off while in use. The coupling mechanism must provide for efficient transfer of power from the drive mechanism of the personal care device to the attachment for optimal operation of the attachment. Additionally, the coupling mechanism must not be too loose, or the attachment will vibrate out of phase with the drive shaft in operation, causing rattling or other noise that impacts on the user experience.

Therefore, according to certain embodiments of the disclosure, a coupling mechanism for coupling first and second components of a personal care device 10 is provided that makes use of material that can change shape with changes in temperature, and the material is configured or arranged in the first component in such a way as to allow a change in temperature of the material to enable the coupling of the first component to the second component. Thus, a coupling interface of a first component of a personal care device 10 can comprise one or more portions of material that can change shape with changes in temperature, and the personal care device 10 has a heating and/or cooling element that can be used to selectively heat and/or cool the portion of material to cause it to change shape and enable the coupling of the coupling interface of the first component of the personal care device to a coupling interface of a second component of the personal care device. The coupling of the first component to the second component enables motion, vibration and/or torque to be transferred between the first component and the second component. This coupling mechanism can be used instead of the coupling mechanism shown in the exemplary device in Figs. 2 and 3, or used in combination.

In some embodiments the first component (i.e. the component that comprises the portion of material) is the attachment structure 16 of the personal care device 10, for example a toothbrush head or a shaver cutting element, and the second component is the handle portion 12 of the personal care device 10. In these embodiments, either the handle portion 12 can comprise the heating or cooling element that causes the change in temperature of the portion of material, or the heating or cooling element can be part of the attachment structure 16, in which case the handle portion 12 can be conventional.

In other embodiments, the first component (i.e. the component that comprises the portion of material) is the handle portion 12 of the personal care device 10 and the second component is the attachment structure 16 of the personal care device 10, such as a toothbrush head or a shaver cutting element. In these embodiments, either the attachment structure 16 can comprise the heating or cooling element, or the handle portion 12 can comprise the heating or cooling element (in which case the attachment structure 16 can be conventional).

Use of materials that change shape based on temperature in the coupling mechanism enables a coupling interface of one of the components (such as a recess in a part of an attachment structure 16) to, in some embodiments, better conform to the size and shape of a coupling interface of the other component (such as drive shaft 22), than existing coupling mechanisms, thus providing a better fit so that any rattling (i.e. relative movement of the first component and the second component) can be eliminated or substantially eliminated.

Furthermore, the portion of material can be configured such that a change in temperature can also be used to easily decouple the components from each other. Thus heating or cooling the portion of material can enable the portion of material to change shape and allow the first coupling interface and the second coupling interface to be separated. In this way, the force needed to attach and detach is reduced, but the holding force is still high enough to transfer torque, motion and/or vibration and avoid run-off

The portion of material is arranged in a coupling interface of the first component in such a way that it interacts with a coupling interface of the second component when the first and second components are brought together for coupling. The material can be heated and/or cooled before the components are brought together to enable the coupling, or once the first and second components are in contact with each other.

Various different types of material, with different responses to changes in temperature, can be used in the coupling mechanism. According to the present invention, the material is such that a temperature change causes the material to change from a solid/stiff state to a more pliable (e.g. rubbery or deformable) state where its stiffness is lowered. This enables the portion of material in the coupling interface of the first component to mold or deform to the shape of the coupling interface of the second component. In these embodiments of the present invention , the action of bringing the coupling interfaces together can lead to the deformation of the material. When the temperature change is reversed, the portion of material returns to the solid/stiff state, providing a force that acts to 'clamp' the coupling interfaces (and thus the first and second components) together. To decouple the first and second components, the temperature of the material can be changed again such that the material is in its pliable state, which allows the components to be separated. Examples of materials that can be used in these embodiments include phase change materials or thermoplastic polymers, such as poly-lactic acid, perfluorosulphonic acid ionomer, polyesterurethane, polyurethane, and poly($\varepsilon$-caprolactone)dimethacrylate.

In other embodiments of the present invention, the material can be such that a temperature change causes the material to change from a solid state to a liquid state. This enables the portion of material in the coupling interface of the first component to flow around, and to adapt to the shape of, the coupling interface of the second component. When the temperature change is reversed, the portion of material returns to a solid state, and thus acts to grip the coupling interface of the second component. To decouple the first and second components, the temperature of the material can be changed again so that the material is in the liquid state, which allows the components to be separated. Examples of materials that can be used in these embodiments include phase change materials (e.g. a wax) or thermoplastic polymers, such as Parrafin, Camphenilone, Caprylone, Methyl eicosanate, Acrylic acid, Glycerin, Methyl palmitate, Capric acid, Polyethylene Glycol, $Mn(NO_3)2.6H_2O$, and $Na_2CO_3.10H_2O$.

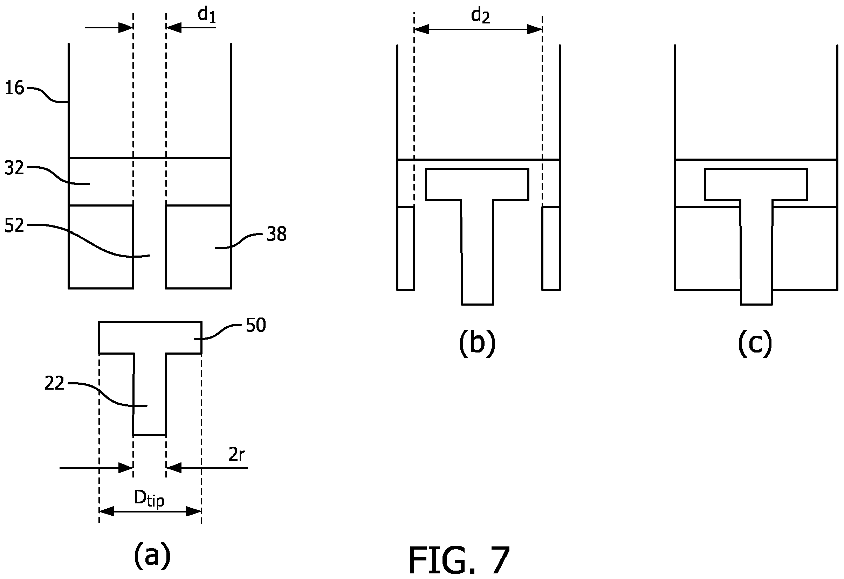

In other embodiments, the material can be such that a temperature change itself causes a change in shape of the material, that is the shape of the material is temperature-dependent. For example, heating the material could cause the material to expand by a certain amount (based on the temperature change), which allows the first and second components to be brought together, and cooling the material could cause the material to contract by a certain amount (again based on the temperature change), thereby coupling the components together. To decouple the first and second components, the temperature of the material can be changed again so that the material expands to allow the components to be separated. An example of a coupling mechanism formed using a material of this type is described below with reference to Fig. 7. In these embodiments the material can be aluminum, copper, polyester, silicone rubber, or epoxy.

In some embodiments the material can be a shape memory material, such as a shape memory polymer. Shape memory materials are materials that can be deformed when heated above a glass transition temperature Tg and will become stiff and retain their new (i.e. deformed) shape when cooled (provided of course that the deforming force is present during cooling). When cooled, the shape memory materials apply force to a coupling interface of a second component. When the shape memory material is reheated above the glass transition temperature Tg, and the deforming force is removed, the shape memory material recovers its original shape. Thus a coupling mechanism having a shape memory material suitable to be re-used in subsequent similar coupling/decoupling cycles. Some shape memory materials or polymers can be deformed above 55°C, and thus revert to their original shape once reheated above 55°C. Other shape memory materials exist with lower transition temperatures, e.g. Nitinol.

There are several materials which have a shape memory effect, including shape memory polymers such as polynorbornene, certain thermoplastic block copolymers such as polyurethanes, block copolymers of polyethylene terephthalate (PET) and polyethyleneoxide (PEO) or block copolymers containing polystyrene and poly(1,4-butadiene), and an ABA triblock copolymer made from poly(2-methyl-2-oxazoline) and polytetrahydrofuran and crosslinked polyurethanes and PEO derivatives. Other polymers include stretched thermoplastics, which include well known example of stretched Poly-lactic acid (PLA). This material can be deformed when heated above about 55 °C and will recover its original shape when re-heated above that temperature.

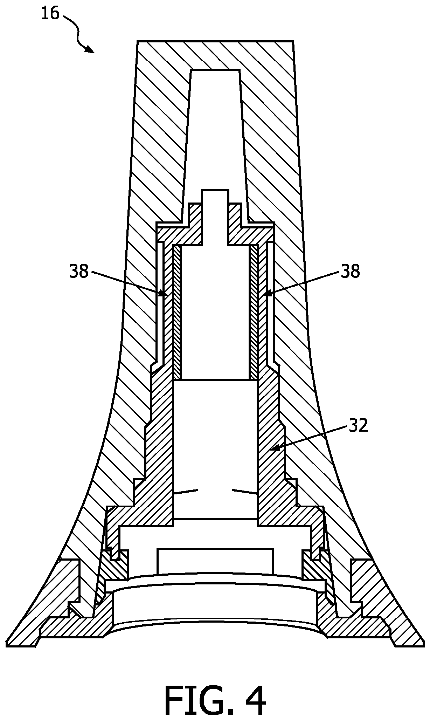

Fig. 4 shows a cut-away side view illustration of an attachment structure 16 according to an embodiment. Fig. 4 shows the coupling interface 32 with a portion of material 38 on the inner surface of the coupling interface 32. The portion of material 38 is positioned such that it can interact (i.e. contact) the drive shaft 22 of the handle portion 12 when the attachment structure 16 is placed over the drive shaft 22 and a temperature change occurs.

As in a conventional attachment structure 16, the coupling interface 32 is generally shaped to receive the drive shaft 22 and to couple the attachment structure 16 to the drive shaft 22 so that a mechanical stimulus from the drive shaft 22 is transmitted to the attachment structure 16.

A heating or cooling element can be provided in the attachment structure 16 (although it is not shown in Fig. 4), or, more preferably, the handle portion 12 can be adapted so that the drive shaft 22 acts as the heating or cooling element for the material 38.

In some embodiments, the size and/or position of the portion of material 38 is such that the remaining space in the coupling interface 32 is slightly smaller than the minimum drive shaft size. In other words, the diameter of the opening remaining at the top of the coupling interface 32 where the material 38 is located is slightly less than the diameter of the drive shaft 22. In this embodiment, the material 38 can be such that the stiffness of the material 38 changes with changes in temperature. In this case, the material 38 can be heated as the drive shaft 22 is inserted such that the material 38 is pliable and can change shape (e.g. through deformation) to enable the drive shaft 22 to be fully inserted. When the material 38 is cooled back to the original temperature (e.g. room temperature) it is no longer pliable and the material 38 exerts a pressing force and friction on the drive shaft 22 to couple the attachment structure 16 to the handle portion 12.

In other embodiments, the size and/or position of the portion of material 38 is such that the drive shaft 22 can be readily inserted into the coupling interface 32, i.e. the remaining space in the coupling interface 32 is larger than the diameter of the drive shaft 22. In this embodiment, the material 38 can be such that the material changes to a liquid state when heated. In this case, the material 38 can be heated once the drive shaft 22 is inserted and the material 38 can flow around the drive shaft 22, before being cooled to retain its shape.

In some embodiments of the present invention, , particularly where the material 38 is a material that changes into a liquid state on heating, it is necessary to contain the material 38 in some way to prevent leakage of the material from the attachment structure 16. Thus, the material 38 can be contained within a flexible film, for example a plastic film that enables the material 38 to flow in a liquid form and adapt to the shape of the drive shaft 22 when the material 38 is heated. In some embodiments, the flexible film may be adapted to encourage the portion of material 38 to revert to an original shape when the material 38 is re-heated and the attachment structure 16 is to be decoupled from the drive shaft 22.

In some embodiments of the present invention, the drive shaft 22 is shaped so as to comprise one or more grooves and/or protrusions. Thus, when the portion of material 38 is heated and the drive shaft 22 is inserted, the material 38 deforms to at least partially fill the grooves and/or at least partially surround the protrusions, thus improving the mechanical coupling and friction between the attachment structure 16 and the drive shaft 22.

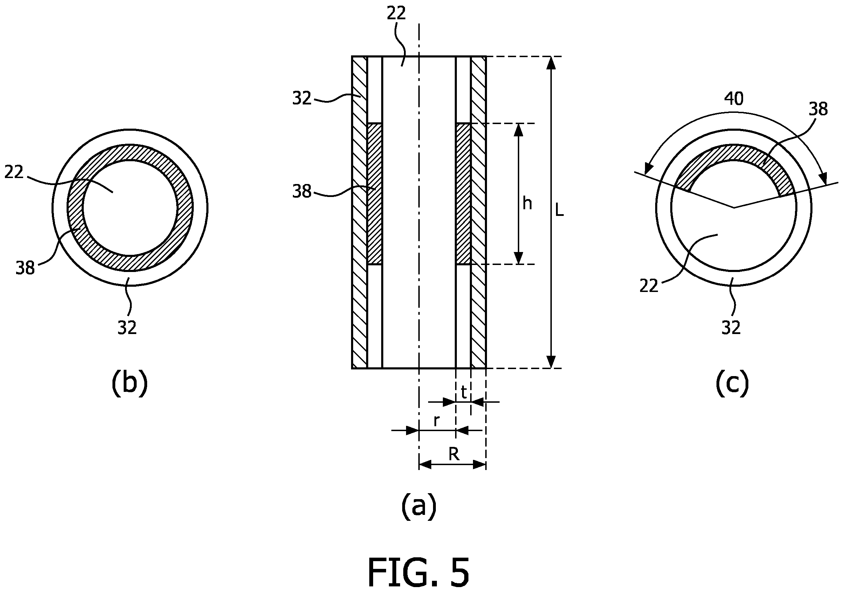

Fig. 5 shows three cross sections through part of the attachment structure 16 of Fig. 4 with a drive shaft 22 inserted in the coupling interface 32. Fig. 5(a) shows a cross-sectional side view through the attachment structure 16 and drive shaft 22, and Figs. 5(b) and 5(c) show axial cross-sections of an attachment structure 16 and drive shaft 22.

As shown in Fig. 5(b), the material 38 lines the full circumference of the inside of the coupling interface 32. The portion of material 38 has a height h as shown in Fig. 5(a), and a thickness t. The drive shaft 22 has a radius r. The length of the coupling interface is L. Fig. 5(c) shows that the effective clamping area of the material 38 on the drive shaft 22 can be adapted by not using the full circumference, but only part of it as indicated by angle 40.

As noted above, in some embodiments, the attachment structure 16 is clamped onto the drive shaft 22 by having an overclosure or overlap between the material 38 and the drive shaft 22. Where the material 38 is a phase change material that changes to a rubbery state on heating, inserting the drive shaft 22 into the attachment structure 16 the material 38 will be compressed and will generate a normal force onto the drive shaft 22 which provides a friction force that keeps the attachment structure 22 in place. An analytical expression for the clamping force Fc can be derived as follows:

$$F_c \ = \mu \ F_n \ = \mu \sigma_i \ A_i \ = \mu \ \frac{\delta t \ E}{r_i \ln\left(\frac{r_o}{r_i}\right)} \ 2\pi \ r_i \ h$$

where $\mu$ is the friction coefficient, $\delta t$ is the overclosure in mm, $F_n$ the normal force, $\sigma$ the stress, $A$ the area, r the radius, $h$ the height of the clamping material and E the modulus of elasticity in MPa. The subscripts i and o denote the inner and outer diameter of the clamping material. A simulation for the case of t = 0.9 mm, E = 3.5 GPa (which is typical for polylactic acid below its glass transition temperature Tg), v = 0.33, ri = 1.5 mm, and t0 = 1.0 mm provides radial stress of 460.7 MPa and 456.8 MPa at the inner wall of the coupling interface 32, which is in agreement with the analytical expression. With a friction coefficient of $\mu$ = 0.25 (typical of POM (Polyoxymethylene) on steel, a possible material for the drive shaft 22), and a height of h = 10 mm, the friction force is 10762 Newton, N.

A typical force needed to retain an attachment structure 16 would be around 20N. This value can be readily achieved by selecting an appropriate combination of contact area, thickness and overclosure for a material 38 with given modulus and friction coefficient. As noted above, a possible material to be used for the clamping would be PLA (polylactic acid). Typical values for the modulus are in the range of 3.5 GPa. The material has a glass transition temperature Tg around 55-65 °C after which the modulus drops into the MPa range, an order of 10 lower. This property can be used to lower the clamping force during attachment and detachment of the attachment structure 16, while achieving a very high clamping force during usage of the personal care device 10, simply by heating the clamping material above its glass transition temperature Tg during attachment and detachment. Since the modulus relates linearly to the clamping force, clamping force differences in the order of 10 can be achieved. In order to exploit the reduction of storage modulus above the glass transition temperature Tg the material 38 needs to be heated up. This should be accomplished in a reasonable amount of time and in a controlled way and can be achieved by a heating element that is temperature controlled at a constant value of about 80 °C.

In some embodiments, to improve the speed with which the material 38 is heated, the thermal conductivity of the portion of material 38 can be adjusted by making use of fillers, such as graphite, metallized glass, or aluminum fibers.

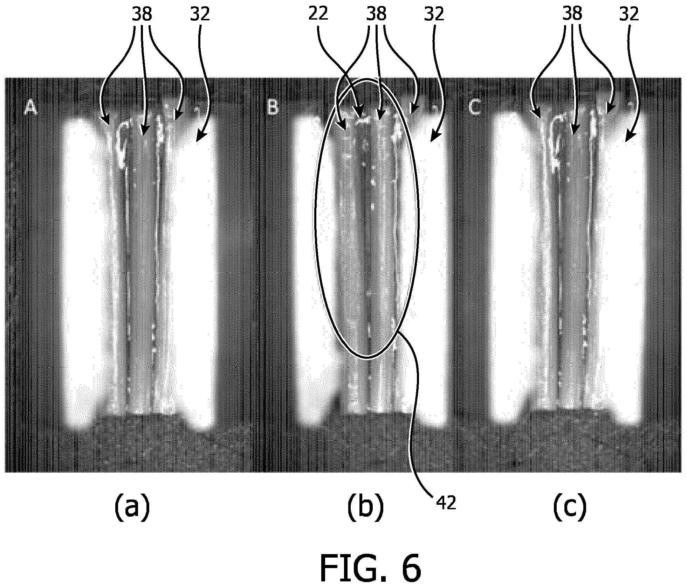

Figs. 6a, 6b and 6c show an example of a coupling mechanism that uses a material having a shape memory effect. In particular, the inside of an attachment structure 16 that has three portions of poly-lactic acid (PLA) 38 arranged on the inside of the coupling interface 32. As noted above, PLA is a material that deforms when heated under load and recovers its original shape when it is heated without loading. A handle portion 12 of a personal care device 10 has a drive shaft 22 modified to be heated and used to heat the attachment structure 16 from the inside to allow deformation of the PLA 38. Fig. 6(a) shows the PLA 38 before heating and before the drive shaft 22 is inserted into the coupling mechanism 32, Fig. 6(b) shows the PLA 38 after heating and insertion of the drive shaft 22 (where it can be seen that some deformation of the PLA 38 has occurred). The PLA 38 can then be cooled (i.e. the drive shaft 22 can cease heating the PLA 38), and the PLA 38 can retain the deformed shape and apply force to the drive shaft 22. This force holds the drive shaft 22 in place and allows the motion, vibration and/or torque of the drive shaft 22 to be transferred to the attachment structure 16. The indents in the PLA 38 due to the insertion of the drive shaft 22 are visible in Fig. 6(b) and marked with circle 42. When the attachment structure 16 is to be decoupled from the handle portion 12, the drive shaft 22 applies heat to the PLA 38 so that it becomes deformable, enabling the drive shaft 22 to be removed from the attachment structure 16. Due to the shape memory properties of the PLA 38, the PLA 38 returns to its original shape almost straight away after the drive shaft 22 is removed (while the PLA 38 is still above the required temperature). Fig. 6(c) shows the PLA 38 after re-heating and recovery of the original shape. Thus, after re-heating, the indents in the PLA 38 have disappeared because the shape memory material has reverted to its original state.

Figs. 7a, 7b and 7c show an example of an alternative coupling mechanism in which the portion of material 38 is used to 'lock' a drive shaft 22 inside an attachment structure 16. In particular, Fig. 7a shows a cross-section through a coupling interface 32 of an attachment structure 16 and a drive shaft 22. One or more portions of material 38 are arranged in the coupling interface 32. The material 38 is such that it contracts on heating, and expands on cooling, for example Cubic Zirconium Tungstate ($ZrW_2O_8$) or engineered composite metamaterials. In this embodiment, the drive shaft 22 is shaped so as to have a tip portion 50 at the tip of the drive shaft 22 that is wider than the rest of the drive shaft 22. This portion 50 has a diameter Dtip, and the diameter of the rest of the drive shaft 22 is 2r. In this embodiment, the portion of material 38 is configured in the attachment structure 16 such that the opening 52 into the attachment structure 16 when the material 38 is at room temperature has a diameter d1. This diameter d1 is less than Dtip which means that the drive shaft 22 cannot be fully received in the coupling interface 32. Upon heating of the portion of material 38 when the drive shaft 22 is to be inserted (which can be achieved by the tip 50 of the drive shaft 22 heating the material 38), the portion of material 38 may change shape (contract) such that the diameter of the opening 52 into the coupling interface 32 (d2) is larger than the diameter of the tip 50 of the drive shaft 22 (i.e. d2 > Dtip), and thus the drive shaft 22 can be received in the coupling interface 32 of the attachment structure 16, as shown in Fig. 7(b). Subsequently, as shown in Fig. 7(b), the material 38 can be cooled back to room temperature so that it reverts to its original shape and size, thus trapping the tip 50 of the drive shaft 22 within the attachment structure 16. Furthermore, in embodiments where the diameter d1 of the opening into the coupling interface 32 at room temperature is less than the diameter of the rest of the drive shaft 22 (i.e. d1 < 2r), cooling the material 38 to room temperature after insertion of the drive shaft 22 will result in the material 38 exerting a compression force on the drive shaft 22, further improving the coupling of the drive shaft 22 to the attachment structure 16.

In any of the above embodiments, a conventional attachment structure retaining system may be used in combination with the portion of material 38 to assist in the coupling and take up any thermal shrinkage of the portion of material 38, and prevent decoupling of the attachment structure 16 during use of the device 10. In some embodiments, this conventional retaining system can be a spring-clip based retaining system, as shown in Fig. 3. As noted above, in a spring-clip based retaining system, one or more clips/coupling springs 33 are provided in the coupling interface 32 that are configured to interface with a protrusion or indentation on the drive shaft 22 when the attachment structure 16 is attached to the drive shaft 22.

In the above embodiments, any suitable heating or cooling element can be used to effect the temperature change of the portion of material 38, and the heating/cooling element can be part of the attachment structure 16, or part of the handle portion 12. An exemplary heating element is a resistive heater, and an exemplary cooling element is a Peltier element. In the embodiments where the drive shaft 22 of a handle portion 12 is used to cause the temperature change, it will be appreciated that a heating or cooling element can be provided in the body of the handle portion 12 that heats or cools the drive shaft 22 as required. In this case, the drive shaft 22 can be formed from a metal or other material that conducts heat in order to transfer the heat/cool from the element to the material 38. The heating/cooling element can be activated and deactivated using a button or switch on the personal care device 10, for example a button or switch that is to be pressed in order to couple and/or decouple the components of the device 10.

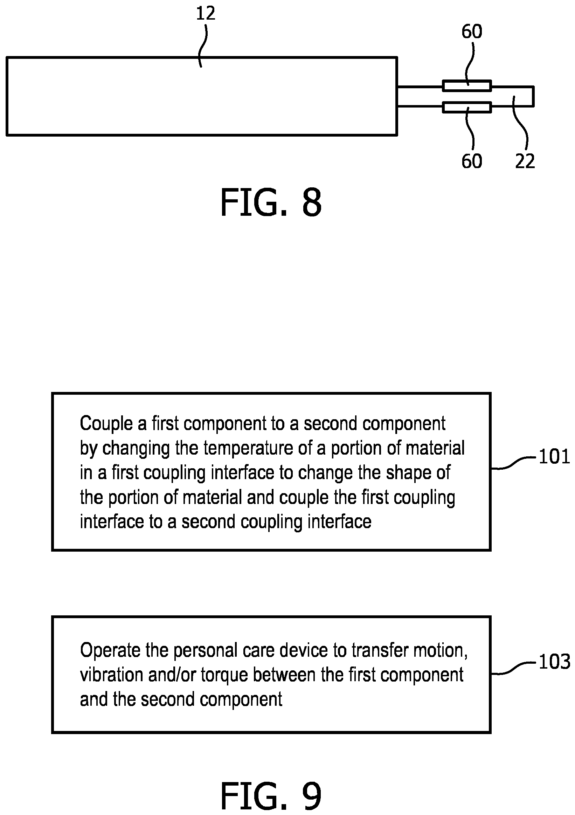

In the above embodiments the portion of material is part of the attachment structure 16. However, in alternative embodiments the portion of material can be provided on the handle portion 12. In particular, as shown in Fig. 8, one or more portions of material 60 can be provided on or in the drive shaft 22 of the handle 12. The handle 12 is generally as described above with reference to Fig. 2, and the drive shaft 22 has one or more portions of material 60 attached thereto or integrated into the drive shaft 22. In this illustrated embodiment two portions 60 are shown, but it will be appreciated that more or less portions 60 can be used. The portions 60 are positioned or arranged on the drive shaft 22 such that they can press against the interior surface of the coupling interface of an attachment structure, e.g. attachment structure 2 in Fig. 1 after a change in temperature of the material 60 in order to couple the handle 12 to an attachment structure. As noted above, the drive shaft 22 may act as a heating and/or cooling element (not shown in Fig. 8) to selectively heat and/or cool the portion of material 60. In this embodiment, if the heating/cooling element is provided in the handle 12, the handle 12 can be coupled with conventional attachment structures 2, i.e. attachment structures that do not comprise a portion of material 38.

As described above, the personal care device 10 comprises a first component and a second component that arc to be coupled together, for example an attachment structure 16 and a handle 12. The first component comprises a body having a first coupling interface (e.g. coupling interface 32), and the second component comprises a second coupling interface (e.g. drive shaft 22). The first component also comprises a coupling interface (a 'first' coupling interface) having a portion of material 38 that changes shape with changes in temperature.

In a first step 101, a method of operating a personal care device 10 according to an exemplary embodiment comprises coupling the first component to the second component by changing the temperature of the portion of material 38 in the first coupling interface. This change in temperature (for example heating or cooling) changes the shape of the portion of material 38 and enables the coupling of the first coupling interface to a second coupling interface. This change in temperature can also or alternatively change the stiffness of the portion of material. The change in the properties of the portion of material in response to the change in temperature allows for easy coupling of the first coupling interface to the second coupling interface.

Next, the method comprises, a step 103 of operating the personal care device 10 such that motion, vibration and/or torque is transferred between the first component and the second component.

In some embodiments, the method further comprises decoupling the first component from the second component by changing the temperature of the portion of material 38. This change in temperature (which can be a similar change in temperature to that used in step 101) can change the shape of the portion of material 38 and enable the first coupling interface to be decoupled from the second coupling interface.

In some embodiments, the first step can comprise heating the portion of material 38 so that it is deformable, deforming the portion of material 38, and cooling the portion of material 38 such that the portion of material 38 retains a deformed shape. Deforming the portion of material 38 comprises deforming the portion of material 38 around at least part of the second coupling interface, for example a drive shaft 22.

In some embodiments, the first step can comprise a first one of heating and cooling the portion of material 38 to change the shape of the portion of material and allow the second coupling interface to be received by the first coupling interface, and the other one of heating and cooling the portion of material to change the shape of the portion of material and trap (i.e. prevent removal of) part of the second coupling interface within the first coupling interface. This embodiment is similar to that shown in Fig. 7. The method can further comprise decoupling the first component from the second component by the first one of heating and cooling the portion of material to change the shape of the portion of material 38 so as to release the part of the second coupling interface from the first coupling interface.

In some embodiments, the first step can also comprise strengthening or improving the coupling connection by changing the temperature of the portion of material to revert the shape or stiffness of the material back to an original or initial state.

Embodiments provide a replaceable toothbrush head that can be easily attached and detached and has a high holding force. The handle has a shaft that can be heated and the brush head contains a material that easily deforms above a transition temperature and is stiff below that temperature. The material is heated during attachment and detachment of the brush head, thereby facilitating easy removal (i.e. a good user experience), while during brushing, it has a high holding force to avoid run-off of the brush during use. Preferably, the coupling interface is adaptive so that the force needed to attach and detach is reduced but the holding force is high enough to transfer toque and avoid run-off. This can for instance be achieved by applying a heat activated shape changing material in the brush head and heating the shaft to activate the shape change. In the claims, the notion "revert *towards* an original shape after the first coupling interface and the second coupling interface have been coupled" does not necessarily imply that the original shape is actually fully achieved after the first coupling interface and the second coupling interface have been coupled. When the shaft is still inserted, the material will try to revert back to its original shape. However, the presence of the shaft may prevent the material from fully reverting to the original shape so that the material may therefore stay in some deformed shape because of the shaft. In embodiments, as a result of this mechanism, the high holding force is achieved.

In addition, any reference signs placed in parentheses in one or more claims shall not be construed as limiting the claims. The word "comprising" and "comprises," and the like, does not exclude the presence of elements or steps other than those listed in any claim or the specification as a whole. The singular reference of an element does not exclude the plural references of such elements and vice-versa.

## Claims

**1.** A first component (16; 12) for a personal care device (10), the first component comprising a first coupling interface for allowing the first component to be releasably and repeatably coupled to a second component (12; 16) for the personal care device via a second coupling interface of the second component,
**characterized in that** the first coupling interface comprises a portion of material (38; 60) that can change from a solid or stiff state to a more pliable state with a selected change in temperature implemented by a temperature changing element of the first or second component (12;16) so that the portion of material (38; 60) is deformable and enables the coupling and uncoupling of the first coupling interface and second coupling interface,
and **in that**, during coupling, the portion of material (38;60) molds or deforms to the shape of the second coupling interface, and reversing the change in temperature changes the portion of material (38;60) from the more pliable state to the solid or stiff state to provide a force to clamp the first coupling interface and the second coupling interface together.

**2.** A first component as in claim 1, wherein the portion of material (38; 60) is configured to provide the force to clamp the first coupling interface to the second coupling interface such that removal of the second coupling interface from the first coupling interface is substantially prevented.

**3.** A first component as in claim 1 or 2, wherein the portion of material (38; 60) is configured to change from the solid or stiff state to the more pliable state with a change in temperature so that the portion of material is deformable and enables the first coupling interface to be decoupled from the second coupling interface.

**4.** A first component as in any one of the preceding claims, wherein the portion of material (38; 60) is configured to be heated to change from the solid or stiff state to the more pliable state so as to allow the second coupling interface to be received by the first coupling interface.

**5.** A first component as in claim 4, wherein the portion of material (38; 60) is configured to be cooled to change from the more pliable state to the solid or stiff state and trap part of the second coupling interface within the first coupling interface.

**6.** A first component as in claim 4 or 5, wherein the portion of material (38; 60) is configured to be heated to change from the solid or stiff state to the more pliable state so as to enable the release of the part of the second coupling interface from the first coupling interface.

**7.** A first component as in any one of claims 1 to 3, wherein the portion of material (38; 60) is configured to be cooled to change from the solid or stiff state to the more pliable state so as to allow the second coupling interface to be received by the first coupling interface.

8. A first component as in claim 7, wherein the portion of material (38; 60) is configured to be heated to change from the more pliable state to the solid or stiff state and trap part of the second coupling interface within the first coupling interface.

9. A first component as in claim 7 or 8, wherein the portion of material (38; 60) is configured to cooled to change from the solid or stiff state to the more pliable state so as to enable the release of the part of the second coupling interface from the first coupling interface.

10. A first component as in any one of claims 1 to 6, wherein the portion of material (38; 60) changes from the solid or stiff state to a liquid state on heating, and wherein the portion of material (38; 60) is contained within a flexible film that is configured to prevent leakage of the portion of material from the first coupling interface when the portion of material is heated.

11. A first component as in claim 10, wherein the flexible film is further configured to return the portion of material to an original shape when the first coupling interface is to be decoupled from the second coupling interface.

12. A first component as in any one of the preceding claims, wherein one of the first coupling interface and the second coupling interface is a drive shaft (22) of a handle (12) of the personal care device (10), and the other one of the first coupling interface and the second coupling interface is part (32) of an attachment (16) for the personal care device.

13. A personal care device (10) comprising:

    the first component (16; 12) as claimed in any one of claims 1 to 12; and
    the second component (12; 16) having the second coupling interface.

14. A personal care device as in claim 13, wherein the second coupling interface comprises one or more grooves and/or protrusions, and wherein the portion of material (38; 60) is configured to be deformable with a change in temperature to at least partially fill the grooves and/or at least partially surround the protrusions and to couple the first coupling interface to the second coupling interface.

15. A personal care device as in claim 13, wherein the second component comprises a temperature changing element configured to selectively change the temperature of the portion of material in the first component.


**Patentansprüche**

1. Erste Komponente (16; 12) für eine Körperpflegevorrichtung (10), wobei die erste Komponente eine erste Kopplungsschnittstelle umfasst, um zu ermöglichen, dass die erste Komponente über eine zweite Kopplungsschnittstelle der zweiten Komponente lösbar und wiederholbar mit einer zweiten Komponente (12; 16) für die Körperpflegevorrichtung gekoppelt werden kann;
   dadurch gekennzeichnet, dass die erste Kopplungsschnittstelle einen Materialabschnitt (38; 60) umfasst, der mit einer ausgewählten Temperaturänderung, die durch ein Temperaturänderungselement der ersten oder zweiten Komponente (12; 16) implementiert wird, von einem festen oder steifen Zustand in einen biegsameren Zustand wechseln kann, so dass der Materialabschnitt (38; 60) verformbar ist und das Koppeln und Entkoppeln der ersten Kopplungsschnittstelle und der zweiten Kopplungsschnittstelle ermöglicht,
   und dass sich während des Koppelns der Materialabschnitt (38; 60) in die Form der zweiten Kopplungsgrenzfläche formt oder verformt, und durch Umkehren der Temperaturänderung wird der Materialabschnitt (38; 60) vom biegsameren Zustand in den festen oder steifen Zustand geändert, um eine Kraft zum Zusammenklemmen der ersten Kopplungsschnittstelle und der zweiten Kopplungsschnittstelle bereitzustellen.

2. Erste Komponente nach Anspruch 1, wobei der Materialabschnitt (38; 60) konfiguriert ist, um die Kraft bereitzustellen, um die erste Kopplungsschnittstelle an der zweiten Kopplungsschnittstelle festzuklemmen, so dass das Entfernen der zweiten Kopplungsschnittstelle von der ersten Kopplungsschnittstelle im Wesentlichen verhindert wird.

3. Erste Komponente nach Anspruch 1 oder 2, wobei der Materialabschnitt (38; 60) konfiguriert ist, um mit einer Änderung der Temperatur vom festen oder steifen Zustand in den biegsameren Zustand zu wechseln, so dass der Materialabschnitt verformbar ist und die Entkopplung der ersten Kopplungsschnittstelle von der zweiten Kopplungsschnittstelle ermöglicht.

**4.** Erste Komponente nach einem der vorhergehenden Ansprüche, wobei der Materialabschnitt (38; 60) so konfiguriert ist, dass er erwärmt wird, um vom festen oder steifen Zustand in den biegsameren Zustand zu wechseln, so dass die zweite Kopplungsschnittstelle von der ersten Kopplungsschnittstelle empfangen werden kann.

**5.** Erste Komponente nach Anspruch 4, wobei der Materialabschnitt (38; 60) so konfiguriert ist, dass er gekühlt wird, um vom biegsameren Zustand in den festen oder steifen Zustand zu wechseln und einen Teil der zweiten Kopplungsschnittstelle innerhalb der ersten Kopplungsschnittstelle einzufangen.

**6.** Erste Komponente nach Anspruch 4 oder 5, wobei der Materialabschnitt (38; 60) so konfiguriert ist, dass er erwärmt wird, um vom festen oder steifen Zustand in den biegsameren Zustand zu wechseln, um die Freigabe des Teils der zweiten Kopplungsschnittstelle von der ersten Kopplungsschnittstelle zu ermöglichen.

**7.** Erste Komponente nach einem der Ansprüche 1 bis 3, wobei der Materialabschnitt (38; 60) so konfiguriert ist, dass er gekühlt wird, um vom festen oder steifen Zustand in den biegsameren Zustand zu wechseln, um zu ermöglichen, dass die zweite Kopplungsschnittstelle von der ersten Kopplungsschnittstelle empfangen wird.

**8.** Erste Komponente nach Anspruch 7, wobei der Materialabschnitt (38; 60) so konfiguriert ist, dass er erwärmt wird, um vom biegsameren Zustand in den festen oder steifen Zustand zu wechseln und einen Teil der zweiten Kopplungsschnittstelle innerhalb der ersten Kopplungsschnittstelle einzufangen.

**9.** Erste Komponente nach Anspruch 7 oder 8, wobei der Materialabschnitt (38; 60) so konfiguriert ist, dass er abkühlt wird, um vom festen oder steifen Zustand in den biegsameren Zustand zu wechseln, um die Freigabe des Teils der zweiten Kopplungsschnittstelle von der ersten Kopplungsschnittstelle zu ermöglichen.

**10.** Erste Komponente nach einem der Ansprüche 1 bis 6, wobei der Materialabschnitt (38; 60) beim Erhitzen vom festen oder steifen Zustand in einen flüssigen Zustand wechselt, und wobei der Materialabschnitt (38; 60) in einem flexiblen Film enthalten ist, der konfiguriert ist, um ein Austreten des Materialabschnitts von der ersten Kopplungsschnittstelle zu verhindern, wenn der Materialabschnitt erwärmt wird.

**11.** Erste Komponente nach Anspruch 10, wobei der flexible Film ferner konfiguriert ist, um den Materialabschnitt in eine ursprüngliche Form zurückzubringen, wenn die erste Kopplungsschnittstelle von der zweiten Kopplungsschnittstelle entkoppelt werden soll.

**12.** Erste Komponente nach einem der vorhergehenden Ansprüche, wobei eine der ersten Kupplungsschnittstellen und der zweite Kupplungsschnittstelle eine Antriebswelle (22) eines Griffs (12) der Körperpflegevorrichtung (10) ist, und die andere der ersten Kopplungsschnittstelle und der zweiten Kopplungsschnittstelle Teil (32) eines Aufsatzes (16) für die Körperpflegevorrichtung ist.

**13.** Körperpflegevorrichtung (10), umfassend:

die erste Komponente (16; 12), wie in einem der Ansprüche 1 bis 12 beansprucht; und
die zweite Komponente (12; 16) mit der zweiten Kopplungsschnittstelle.

**14.** Körperpflegevorrichtung nach Anspruch 13, wobei die zweite Kopplungsschnittstelle eine oder mehrere Rillen und/oder Vorsprünge umfasst, und wobei der Materialabschnitt (38; 60) so konfiguriert ist, dass er mit einer Temperaturänderung verformbar ist, um die Rillen zumindest teilweise zu füllen und/oder die Vorsprünge zumindest teilweise zu umgeben und die erste Kopplungsschnittstelle mit der zweiten Kopplungsschnittstelle zu koppeln.

**15.** Körperpflegevorrichtung nach Anspruch 13, wobei die zweite Komponente ein Temperaturänderungselement umfasst, das konfiguriert ist, um die Temperatur des Materialabschnitts in der ersten Komponente selektiv zu ändern.

**Revendications**

**1.** Premier composant (16; 12) pour un dispositif de soins personnels (10), le premier composant comprenant une première interface de couplage pour permettre au premier composant d'être couplé de manière amovible et répétable à un second composant (12; 16) pour le dispositif de soins personnels via une seconde interface de couplage du second composant,

caractérisé en ce que la première interface de couplage comprend une partie de matériau (38; 60) qui peut passer d'un état solide ou rigide à un état plus souple avec un changement sélectionné de température mis en œuvre par un élément de changement de température du premier ou du second composant (12; 16) de sorte que la partie de matériau (38; 60) est déformable et permet le couplage et le découplage de la première interface de couplage et de la seconde interface de couplage,

et en ce que, lors du couplage, la partie de matériau (38; 60) se moule ou se déforme selon la forme de la deuxième interface de couplage, et l'inversion du changement de température modifie la partie de matériau (38; 60) de l'état le plus souple à l'état solide ou rigide pour fournir une force pour serrer ensemble la première interface de couplage et la seconde interface de couplage.

2. Premier composant selon la revendication 1, dans lequel la partie de matériau (38; 60) est configurée pour fournir la force pour serrer la première interface de couplage sur la seconde interface de couplage de telle sorte que le retrait de la seconde interface de couplage de la première interface de couplage soit sensiblement empêché.

3. Premier composant selon la revendication 1 ou 2, dans lequel la partie de matériau (38; 60) est configurée pour passer de l'état solide ou rigide à l'état plus souple avec un changement de température de sorte que la partie de matériau est déformable et permet à la première interface de couplage d'être découplée de la seconde interface de couplage.

4. Premier composant selon l'une quelconque des revendications précédentes, dans lequel la partie du matériau (38; 60) est configurée pour être chauffée pour passer de l'état solide ou rigide à l'état plus souple de manière à permettre à la seconde interface de couplage d'être reçue par la première interface de couplage.

5. Premier composant selon la revendication 4, dans lequel la partie de matériau (38; 60) est configurée pour être refroidie pour passer de l'état plus souple à l'état solide ou rigide et piéger une partie de la seconde interface de couplage à l'intérieur de la première interface de couplage.

6. Premier composant selon la revendication 4 ou 5, dans lequel la partie de matériau (38; 60) est configurée pour être chauffée pour passer de l'état solide ou rigide à l'état plus souple de manière à permettre la libération de la partie de la seconde interface de couplage de la première interface de couplage.

7. Premier composant selon l'une quelconque des revendications 1 à 3, dans lequel la partie de matériau (38; 60) est configurée pour être refroidie pour passer de l'état solide ou rigide à l'état plus souple de manière à permettre à la seconde interface de couplage d'être reçue par la première interface de couplage.

8. Premier composant selon la revendication 7, dans lequel la partie de matériau (38; 60) est configurée pour être chauffée pour passer de l'état plus souple à l'état solide ou rigide et piéger une partie de la seconde interface de couplage à l'intérieur de la première interface de couplage.

9. Premier composant selon la revendication 7 ou 8, dans lequel la partie de matériau (38; 60) est configurée pour être refroidie pour passer de l'état solide ou rigide à l'état plus souple de manière à permettre la libération de la partie de la seconde interface de couplage de la première interface de couplage.

10. Premier composant selon l'une quelconque des revendications 1 à 6, dans lequel la partie de matériau (38; 60) passe de l'état solide ou rigide à un état liquide par chauffage, et dans lequel la partie de matériau (38; 60) est contenue dans un film flexible qui est configuré pour empêcher la fuite de la partie de matériau depuis la première interface de couplage lorsque la partie de matériau est chauffée.

11. Premier composant selon la revendication 10, dans lequel le film flexible est en outre configuré pour ramener la partie de matériau à une forme originale lorsque la première interface de couplage doit être découplée de la seconde interface de couplage.

12. Premier composant selon l'une quelconque des revendications précédentes, dans lequel l'une entre la première interface de couplage et la seconde interface de couplage est un arbre d'entraînement (22) d'une poignée (12) du dispositif de soins personnels (10), et l'autre entre la première interface de couplage et la seconde interface de couplage fait partie (32) d'un accessoire (16) pour le dispositif de soins personnels.

13. Dispositif de soins personnels (10) comprenant:

le premier composant (16; 12) selon l'une quelconque des revendications 1 à 12; et

le second composant (12; 16) ayant la seconde interface de couplage.

14. Dispositif de soins personnels selon la revendication 13, dans lequel la seconde interface de couplage comprend une ou plusieurs rainures et/ou saillies, et dans lequel la partie de matériau (38; 60) est configurée pour être déformable avec un changement de température pour remplir au moins partiellement les rainures et/ou au moins partiellement entourer les saillies et pour coupler la première interface de couplage à la seconde interface de couplage.

15. Dispositif de soins personnels selon la revendication 13, dans lequel le second composant comprend un élément de changement de température configuré pour changer sélectivement la température de la partie de matériau dans le premier composant.

FIG. 1
Prior Art

FIG. 2

FIG. 3

16

FIG. 4

FIG. 5

EP 3 558 158 B1

FIG. 6

(a)

(b)

(c)

FIG. 7

EP 3 558 158 B1

12

60

60    22

# FIG. 8

Couple a first component to a second component
by changing the temperature of a portion of material
in a first coupling interface to change the shape of
the portion of material and couple the first coupling
interface to a second coupling interface

101

Operate the personal care device to transfer motion,
vibration and/or torque between the first component
and the second component

103

# FIG. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009155718 A
- US 20100298851 A
- US 20130125921 A